# EUROPÄISCHE PATENTSCHRIFT

(11) **EP 1 079 771 B1**
(45) Veröffentlichungstag und Bekanntmachung des Hinweises auf die Patenterteilung: **02.01.2003**
(21) Anmeldenummer: 99926407.0
(22) Anmeldetag: 20.05.1999
(51) Int. Cl.: A61F 2/80

(54) **HÜLSE FÜR AMPUTATIONSSTÜMPFE**
SLEEVE FOR AMPUTATION STUMPS
GAINE POUR MOIGNONS D'AMPUTATION

(30) Priorität: 27.05.1998 DE 19823753
(43) Veröffentlichungstag der Anmeldung: 07.03.2001
(73) Patentinhaber: DEKUMED Gesellschaft für Kunststoff- und Medizintechnik mbH, D-83233 Bernau (DE); F.G. Streifeneder KG, 80333 München (DE)
(72) Erfinder: STREIFENEDER, Bernd, D-82256 Fürstenfeldbruck (DE); RICKAUER, Peter, D-83233 Bernau (DE)
(74) Vertreter: Reitzner, Bruno, Dr.
(86) Internationale Anmeldenummer: EP9903598
(87) Internationale Veröffentlichungsnummer: WO99060958

(56) Entgegenhaltungen:
- WO-A-88/00032
- WO-A-92/08425
- WO-A-94/24965
- DE-U- 7 821 156
- FR-A- 2 620 933
- US-A- 2 696 011
- US-A- 3 186 006
- US-A- 3 732 578
- US-A- 5 263 990
- US-A- 5 728 168

## Beschreibung

Die Erfindung betrifft eine Hülse für Amputationsstümpfe aus einem luftdichten kunststoffhaltigen Material mit einem weiteren offenen proximalen (körpernahen) Ende und einem engeren geschlossenen distalen Ende, an welchem ein Kupplungsglied zum Anbringen einer Prothese vorgesehen ist, wobei die Hülse im Bereich ihres distalen Endes in radialer Richtung eine höhere Elastizität hat als in axialer Richtung.

Derartige Hülsen für Amputationsstümpfe sind beispielsweise aus der WO 88/00032 bekannt. Im allgemeinen hat eine solche Hülse die Form eines Kegelstumpfes, wobei das distale Ende vollständig verschlossen und etwas abgerundet ist. Das Material der Hülse besteht aus einem luftdichten Material, im allgemeinen aus Siliconharz, damit sich die Hülse durch Saugwirkung an den Amputationsstumpf anlegen kann. Außerdem ist das Material der Hülse elastisch, wobei zumindest der distale Bereich in Bezug auf die Elastizität anisotrop ist, derart, daß eine hohe Elastizität in radialer Richtung besteht und das Material in axialer Richtung im wesentlichen unelastisch ist. Vorzugsweise erstreckt sich der elastisch anisotrope Teil der Hülse über mindestens einen größeren Teil der distalen Hälfte, z.B. auf mindestens etwa ein Drittel bis über die Hälfte der Länge der Hülse, gemessen vom distalen Ende.

Die anisotropen elastischen Eigenschaften der Hülse können beispielsweise durch Laminieren eines hochelastischen Materials mit einer Schicht aus einem unelastischen Material, z.B. starren Drähten, erreicht werden, die die Elastizität in axialer Richtung der Hülse herabsetzen, ohne daß sie die Elastizität in radialer Richtung wesentlich beeinflussen. Wenn die Hülse auch in axialer Richtung elastisch wäre, so würden die Kräfte auch auf die distalen weichen Teile des Stumpfes übertragen werden oder diese Teile stark deformieren. Durch die Inelastizität in axialer Richtung wird also eine unerwünschte "Kolbenbewegung" des Stumpfes in der Prothese vermieden.

Zum Verbinden der Hülse mit der Prothese dient ein Kupplungsglied, das beispielsweise einen zentralen Vorsprung am Ende der Hülse enthalten kann, an dem die Prothese befestigt wird.

Die elastische Hülse kann zum Anlegen über den Amputationsstumpf vorzugsweise aufgerollt werden, etwa in der Weise, wie es beim Anziehen eines Strumpfes geschieht. Zur Erleichterung des Aufrollens ist das Material der Hülse am freien körpernahen Ende im allgemeinen dünner als am distalen Ende.

Die Hülsen können sowohl als Hülsen für Beinstümpfe als auch für Armstümpfe ausgebildet werden.

Diese bekannten Hülsen haben jedoch den Nachteil, daß ein Verrutschen am Amputationsstumpf in axialer Richtung nicht mit Sicherheit ausgeschlossen werden kann, beispielsweise wenn sich der Amputationstumpf nach dem Anpassen der Hülse im Laufe der Zeit infolge von Muskelschwund verkleinert. In diesem Fall drückt der Amputationsstumpf auf das geschlossene distale Ende der Hülse, was insbesondere dann nachteilig ist, wenn der Röhrenknochen des Amputationsstumpfes nicht wieder mit ausreichend Muskelgewebe umgeben ist.

Diese Nachteile werden erfindungsgemäß dadurch eliminiert bzw. gemildert, daß nur im Bereich des distalen Endes der Hülse ein Dämpfungsglied vorgesehen ist.

Das Dämpfungsglied kann ein elastisches oder plastisches Material umfassen, z.B. einen elastischen Siliconkautschuk, der etwas weicher ist als das Material der Hülse. Als plastisches Material kann man beispielsweise ein in eine Kunststofffolie eingeschweißtes Gel verwenden.

Das Dämpfungsglied kann entweder fest mit dem distalen Ende der Hülse verbunden sein; es kann aber auch lose in die Hülse eingelegt werden, so daß es aus der Hülse herausnehmbar ist. Die Dicke des Dämpfungsgliedes kann den jeweiligen Anforderungen angepaßt werden kann. Bei Verwendung eines Dämpfungsgliedes ist es nicht unbedingt nötig, daß die Hülse genau dem Amputationsstumpf angepaßt wird. So kann beispielsweise bei einem gegebenen Durchmesser der Hülse und einem dickeren Amputationsstumpf eine Dämpfungsglied mit einer größeren Dicke verwendet werden und umgekehrt. Das Dämpfungsglied kann ferner hinsichtlich Härte und Elastizität den zu erwartenden Belastungen angepaßt werden. Beabsichtigt der Prothesenträger beispielsweise eine längere Wanderung in unebenem Gelände, so kann er ein weicheres Dämpfungsglied verwenden.

Nach einer weiteren Ausführungsform der Erfindung kann in das kunststoffhaltige Material ein textiles Material mit einer höheren Elastizität in radialer Richtung als in axialer Richtung eingebaut sein, beispielsweise ein sogenanntes "Stretchgewebe". Bei dieser Ausführungsform wird die Anisotropie der Elastizität nicht durch das Kunststoffmaterial bestimmt, sondern durch das textile Material, was einfacher zu bewerkstelligen ist. Ein weiterer produktionstechnischer Vorteil besteht darin, daß für die Herstellung von Hülsen mit unterschiedlicher Elastizität dasselbe Kunststoff-Ausgangsmaterial verwendet werden kann und eine genaue Einstellung der Polymerisationsbedingungen nicht erforderlich ist. Außerdem hat eine Hülse, in die ein textiles Material eingebaut ist, eine höhere Reißfestigkeit als ein reines Kunststoffmaterial.

Nach einer weiteren Ausführungsform der Erfindung ist das kunststoffhaltige Material im Bereich der Kniekehle bzw. der Armbeugesehne dünnwandiger als in anderen Bereichen, so daß beim Abwinken des Knies bzw. des Arms keine Wülste entstehen.

Das Kupplungsglied ist im allgemeinen fest in das Kunststoffmaterial am distalen Ende der Hülse eingearbeitet. Gewöhnlich enthält es einen Vorsprung in Form einer Pfanne zur Aufnahme eines in der Prothese vorgesehenen Kugelgelenks, wie es z.B. in der WO 88/00032 (Fig. 4a und 4b) beschrieben ist. Das Verbindungsglied kann aber auch ein nach außen ragendes Gewinde zur Aufnahme einer an der Prothese befestigten Schraube enthalten. In diesem Fall wird die Prothese einfach auf die Hülse aufgeschraubt. Im allgemeinen umgreift die Prothese die Hülse, wodurch eine Relativbewegung zwischen Hülse und Prothese ausgeschlossen wird. Eine solche Anordnung ist beispielsweise in der WO 88/00032 (Fig. 4a und 4b) beschrieben.

Die Erfindung ist durch die beigefügte Zeichnung erläutert.
Figur 1 zeigt einen Schnitt durch die Hülse;
Figur 2 zeigt einen vergrößerten Teilschnitt durch die Seitenwand der Hülse im Bereich der Kniekehle;
Figur 3 zeigt einen vergrößerten Teilschnitt durch die Seitenwand in der unteren Hälfte der Hülse.

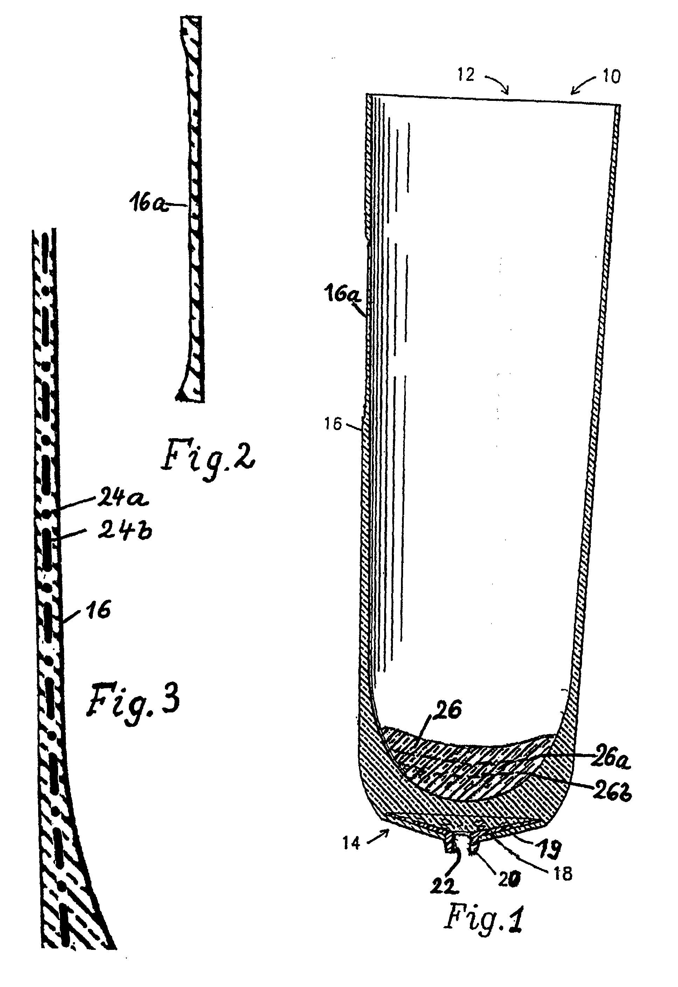

Die Hülse 10 von Figur 1 besteht aus einem elastischen Siliconharz und hat ein offenes proximales (körpernahes) Ende 12 zur Aufnahme eines Amputationsstumpfes (z.B. eines Beinstumpfes), ein geschlossenes distales Ende 14 und eine Seitenwand 16 bestimmter Dicke. Vorzugsweise beträgt die Dicke der Seitenwand etwa 2 mm. An der Stelle 16a (vgl. Figur 2), d.h. an der Stelle, an der bei der Aufnahme des Amputationsstumpfes die Kniekehle zu liegen kommt, beträgt die Dicke nur etwa 1 mm, so daß beim Beugen des Knies kein Wulst entsteht. Weiterhin beträgt die Dicke des Siliconharzes am distalen Ende 14 etwa 10 mm. Am distalen Ende 14 ist ferner ein Kupplungsglied 18 aus einem harten Kunststoff in das Siliconharz eingearbeitet. Das Kupplungsglied 18 ist mit Öffnungen 19 versehen, um eine festere Verbindung zwischen dem Kupplungsglied und dem Siliconharz zu schaffen. Das Kupplungsglied enthält einen aus dem distalen Ende der Hülse herausragenden Stutzen 20 mit einem Innengewinde 22, in das die Prothese (nicht dargestellt) mit einem Außengewinde eingeschraubt wird.

In die Seitenwand 16 ist ein Textilgewebe 24 (vgl. Figur 3) eingebaut, das aus den radial verlaufenden Schußfäden 24a aus einem elastischen Material (z.B. Elasthan) und den axial verlaufenden Kettfäden 24b aus einem starren Material (z.B. Polyamid) besteht. Mit der Bezugszahl 26 ist ein Dämpfungsglied aus einem elastischen Material bezeichnet, das bei der dargestellten Ausführungsform lediglich in die Hülse 10 eingelegt ist. Das Dämpfungsglied 26 besteht aus einem elastischen Siliconharz, das etwas weicher ist als das Material der Hülle 10. Natürlich kann das Dämpfungsglied 26 am distalen Ende der Hülle 10 auch eingeklebt oder anpolymerisiert werden. Die Möglichkeit, das Dämpfungsglied mit unterschiedlicher Dicke auszubilden, ist durch die gestrichelten Linien 26a und 26b angedeutet.

Das Dämpfungsglied 26 kann aber auch als gelartige Masse ausgebildet sein, die in einem Folienbeutel eingeschweißt ist.

## Patentansprüche

1. Hülse (10) für Amputationsstümpfe aus einem luftdichten kunststoffhaltigen Material, mit einem weiteren offenen proximalen Ende (12) und einem engeren geschlossenen distalen Ende (14), an welchem ein Kupplungsglied (18) zum Anbringen einer Prothese vorgesehen ist, wobei die Hülse (10) im Bereich ihres distalen Endes (14) in radialer Richtung eine höhere Elastizität hat als in axialer Richtung, **dadurch gekennzeichnet, daß** nur im Bereich des distalen Endes der Hülse ein Dämpfungsglied (26) vorgesehen ist und daß in das kunststoffhaltige Material ein textiles Material mit einer höheren Elastizität in radialer Richtung (24a) als in axialer Richtung (24b) eingebaut ist.

2. Hülse nach Anspruch 1, **dadurch gekennzeichnet, daß** das Dämpfungsglied (26) ein plastisches oder elastisches Material darstellt.

3. Hülse nach Anspruch 1 oder 2, **dadurch gekennzeichnet, daß** das Dämpfungsglied (26) mit dem distalen Ende der Hülse (10) verbunden oder aus der Hülse (10) herausnehmbar ist.

4. Hülse nach einem der Ansprüche 1 bis 3, **dadurch gekennzeichnet, daß** in das kunststoffhaltige Material im Bereich der Kniekehle bzw. der Armbeugesehne (16a) dünnwandiger ist als in anderen Bereichen.

## Claims

1. Sleeve (10) for amputation stumps made of an air-tight plastic-containing material, with a wider open proximal end (12) and an narrower closed distal end (14) on which a coupling member (18) for the attachment of a prosthesis is provided, the sleeve (10) in the area of its distal end (14) having a higher elasticity in radial direction than in axial direction, **characterized in that**, only in the area of the distal end of the sleeve, an attenuating member (26) is provided and that a textile material having a higher elasticity in radial direction (24a) than in axial direction (24b) is incorporated in the plastic-containing material.

2. Sleeve according to Claim 1, **characterized in that** the attenuating member (26) represents a plastic or elastic material.

3. Sleeve according to Claims 1 or 2, **characterized in that** the attenuating member (26) is connected to the distal end of the sleeve (10), or can be taken out of the sleeve (10).

4. Sleeve according to any one of Claims 1 to 3, **characterized in that** the plastic-containing material has a smaller wall thickness in the area of the knee bend or of the arm bending tendon (16a) than it has in other areas.

## Revendications

1. Fourreau (10) pour moignons de membres amputés réalisé en un matériau étanche à l'air à base de matière plastique, présentant une extrémité proximale ouverte plus large (12) et une extrémité distale fermée plus étroite (14), sur laquelle est prévu un élément d'accouplement (18) pour attacher une prothèse, le fourreau (10) présentant dans la zone de son extrémité distale (14), dans le sens radial, une élasticité plus importante que dans le sens axial, **caractérisé en ce qu'**il est prévu, uniquement dans la zone de l'extrémité distale du fourreau, un élément amortisseur (26) et **en ce qu'**une matière textile présentant une élasticité plus importante dans le sens radial (24a) que dans le sens axial (24b) est incorporée dans le matériau à base de matière plastique.

2. Fourreau selon la revendication 1, **caractérisé en ce que** l'élément amortisseur (26) est constitué d'une matière plastique ou élastique.

3. Fourreau selon la revendication 1 ou 2, **caractérisé en ce que** l'élément amortisseur (26) est relié à l'extrémité distale (10) du fourreau ou peut être retiré du fourreau (10).

4. Fourreau selon l'une quelconque des revendications 1 à 3, **caractérisé en ce que** le matériau à base de matière plastique présente une paroi plus mince dans la zone du creux du genou ou du pli du coude (16a) que dans d'autres zones.
